# EUROPEAN PATENT APPLICATION

(11) **EP 4 043 038 A1**
(43) Date of publication of application: **17.08.2022**
(21) Application number: 20874700.6
(22) Date of filing: 09.10.2020
(51) Int. Cl.: A61K 48/00, C12N 5/10, C12N 15/113, C12N 15/12, C12N 15/13, C12N 15/62, C12N 15/867, A61K 31/7105, A61K 35/12

(54) **SIRNA EXPRESSION VECTOR**

(30) Priority: 11.10.2019 JP 2019187309
(71) Applicant: Takara Bio Inc., Kusatsu-shi Shiga 525-0058 (JP)
(72) Inventor: OKAMOTO, Sachiko, Kusatsu-shi, Shiga 525-0058 (JP); MAKI, Izumi, Kusatsu-shi, Shiga 525-0058 (JP); MINENO, Junichi, Kusatsu-shi, Shiga 525-0058 (JP)
(74) Representative: Müller, Christian Stefan Gerd
(86) International application number: PCT/JP2020/038372
(87) International publication number: WO 2021/070956

(57) **Abstract**

Provided is a nucleic acid construct for expressing a gene of interest, the nucleic acid construct comprising, in order from 5'-terminus, each sequence of: (a) a 5' long terminal repeat (LTR) sequence derived from a retrovirus; (b) a packaging signal sequence (ψ) derived from a retrovirus; (c) a sequence or multiple cloning site of the gene of interest; (d) a post-transcriptional regulatory sequence (PRE); (e) an siRNA generating sequence which forms at least one stem-loop structure and in which RNA, which induces RNA interference in mammalian cells, is transcribed; and (f) a 3' LTR sequence derived form a retrovirus.

## Description

### Technical Field

The present invention relates to a nucleic acid construct that produces an siRNA that induces RNA interference in mammalian cells and expresses a gene of interest, a retroviral vector for introducing the nucleic acid construct into cells, a nucleic acid construct for producing the vector, a method for producing a gene-introduced cell comprising using the vector, and a cell containing the nucleic acid construct.

### Background Art

RNA interference (RNAi) is a phenomenon that can induce destruction of an mRNA encoding a target gene to suppress the expression of the target gene by introducing a double-stranded RNA (hereinafter, referred to as "dsRNA") consisting of a sense RNA and an antisense RNA into cells, wherein the sense RNA consists of a sequence homologous to the mRNA of the target gene and the antisense RNA consists of a sequence complementary to the sense RNA. RNA interference is attracting attention as a simple method for inhibiting gene function or as an applicable method to gene therapy. RNA interference was a phenomenon initially discovered in nematodes (see Non-Patent Literature 1). In the present day, RNA interference is observed in various organisms such as plants, protozoans, insects, and mammals. Suppression of gene expression by RNA interference is called gene knockdown.

Subsequent studies have shown that RNase III, known as dicer, cleaves a dsRNA to produce a siRNA (short interfering RNA) of around 20 bases, and that the siRNA is involved in cleavage of a target RNA. It has been found that the siRNA forming a double strand has a protruding end of 2 or 3 bases on the 3' side and this is important to efficient cleavage of a target RNA (see Non-Patent Literature 2). Such a siRNA binds to a protein complex called RISC (RNA induced silencing complex), and the RISC to which the siRNA binds cleaves an mRNA of a target gene in a sequence-specific manner. The siRNA is believed to suppress the expression of a target gene by such a process.

It has been also reported that an siRNA is generated from a single-stranded short hairpin (hereinafter, referred to as "sh") RNA that forms a stem-loop structure and is effective in inducing RNA interference (Non-Patent Literature 3). Thus, at present, a vector for expressing an shRNA is an important tool for analysis and utilization of RNA interference.

A retroviral vector for producing an siRNA and expressing a gene of interest is reported in Patent Literature 1. However, development of a more efficient vector is demanded.

### Citation List

### Non-Patent Literatures

Non-Patent Literature 1: Nature, vol. 391, pp. 806-811 (1998)
Non-Patent Literature 2: Genes Dev., vol. 15, pp. 188-200 (2001)
Non-Patent Literature 3: Nat. Biotechnol., vol. 23, pp. 227-231 (2005)

### Patent Literatures

Patent Literature 1: WO2012/157742

### Summary of Invention

### Problem to be solved by the Invention

An object of the present invention is to provide a retroviral vector for introducing an exogenous gene into a cell to express the gene, and further bringing about transcription into an RNA that induces RNA interference in the cell to suppress expression of a specific endogenous gene.

### Solution for Problem

As a result of diligent efforts to solve the above problem, the present inventors found a retroviral vector capable of both expressing an introduced gene and suppressing expression of an endogenous gene by RNA interference. Thus the present invention was completed.

That is, the present invention is outlined as follows.
[1] A nucleic acid construct for expressing a gene of interest, comprising: in order from the 5' end to 3' end,
   (a) a 5' LTR (Long Terminal Repeat) sequence derived from a retrovirus,
   (b) a packaging signal sequence (ψ) derived from a retrovirus,
   (C) a sequence of the gene of interest or a multiple cloning site,
   (d) a posttranscriptional regulatory element (PRE),
   (e) a siRNA-producing sequence which is transcribed into an RNA that forms at least one stem-loop structure and induces RNA interference in a mammalian cell, and
   (f) a 3' LTR sequence derived from a retrovirus.
[2] The nucleic acid construct according to [1], wherein the PRE is a PRE derived from Woodchuck hepatitis virus (WPRE).
[3] The nucleic acid construct according to [1], which comprises a foreign promoter sequence on the 5' side of the gene of interest sequence.
[4] The nucleic acid construct according to [1], wherein the gene of interest is a gene encoding a T cell receptor (TCR) or a chimeric antigen receptor (CAR).
[5] The nucleic acid construct according to [4], wherein the gene of interest is a gene encoding a TCRα chain and a TCRβ chain linked with a 2A peptide.
[6] The nucleic acid construct according to [1], wherein the siRNA-producing sequence is a sequence for producing siRNA that acts on a mRNA encoding a constant region of a wild-type TCR to suppress its expression.
[7] The nucleic acid construct according to [6], wherein the gene of interest is a gene encoding a mutated TCR in which a mutation is introduced into a nucleotide sequence of a constant region, and expression of the gene encoding the mutated TCR is not suppressed by the siRNA.
[8] The nucleic acid construct according to [1], wherein the siRNA-producing sequence is a sequence for producing siRNA that acts on a mRNA encoding a molecule that suppresses an activity of an immune cell to suppress its expression.
[9] A retroviral vector comprising a transcript from the nucleic acid construct according to any one of [1]-[8].
[10] The retroviral vector according to [9], which comprises a 5' LTR derived from an oncoretrovirus or a lentivirus, a packaging signal sequence derived from an oncoretrovirus or a lentivirus, and a 3' LTR derived from an oncoretrovirus or a lentivirus.
[11] A method for producing a retrovirus vector, the method comprising a step of introducing the nucleic acid construct according to any one of [1] to [8] into a cell capable of producing a retrovirus particle.
[12] A method for producing a gene-introduced cell, the method comprising a step of introducing the retroviral vector according to claim [9] or [10] into a cell.
[13] The method according to [12], wherein the cell is a T cell, a cell capable of differentiating into a T cell, or a cell population containing the T cell or the cell capable of differentiating into a T cell.
[14] A cell containing the nucleic acid construct according to any one of [1] to [8].
[15] The gene-introduced cell according to [14], wherein the cell is a T cell, a cell capable of differentiating into a T cell, or a cell population containing the T cell or the cell capable of differentiating into a T cell.

### Effects of the Invention

According to the present invention, a nucleic acid construct capable of efficiently expressing a gene of interest and efficiently suppressing expression of a specific endogenous gene, a retroviral vector for introducing the nucleic acid construct into cells, a nucleic acid construct for producing the vector, a method for producing a gene-introduced cell comprising using the vector, and a cell containing the nucleic acid construct are provided. The nucleic acid construct, retroviral vector, and cell are extremely useful for protein production, treatment of diseases by cell therapy, and research and tests for such protein production and treatment.

### Brief Description of Drawings

[FIG. 1] Figure 1 shows the structures of retroviral vectors prepared in Examples.
[FIG. 2] Figure 2 shows the expression levels of endogenous TCR genes with respect to the number of virus copies.
[FIG. 3] Figure 3 shows the expression levels of exogenous TCR genes with respect to the number of virus copies.
[FIG. 4] Figure 4 shows the structures of retroviral vectors prepared in Examples.
[FIG. 5] Figure 5 shows the number of virus copies integrated into a genome by retrovirus vectors.
[FIG. 6] Figure 6 shows the expression levels of endogenous TCR genes with respect to the number of virus copies.

### Mode for carrying out the Invention

As used herein, the term "nucleic acid construct" means a nucleic acid comprising a sequence constructed so as to contain one or more functional units that are not found in nature. The nucleic acid may be a DNA and/or an RNA, and may include a modified nucleic acid. For example, the nucleic acid may be in a circular form, a linear form, a double-stranded form, or a single-stranded form, or in the form of an extrachromosomal DNA molecule (plasmid) or cosmid. The nucleic acid construct may comprise a nucleic acid sequence encoding a gene and optionally a control sequence (e.g., promoter) operably linked (i.e., in such a manner as to control transcription and translation) to the gene. The nucleic acid construct may optionally comprise other regulatory elements, functional sequences, linkers and the like.

As used herein, the term "LTR (Long Terminal Repeat)" refers to a sequence consisting of 100 to 1000 base pairs which is repeatedly present at the both ends of a provirus DNA of a retrovirus, a retrotransposon or the like. The LTR is composed of U3, R, and U5 regions which are involved in transcription of a viral gene, reverse transcription from a viral genome, and integration of a double-stranded DNA synthesized via the reverse transcription into a host DNA. IR sequences (inverted repeat regions) located at the 5' and 3' ends of the provirus are 4 to 20 base pairs in length. The U3 region contains an enhancer sequence and a promoter sequence for transcription.

As used herein, the term "packaging signal sequence" is also referred to as a "psi sequence" or "ψ sequence", and means a non-coding cis-acting sequence required for formation of capsids of retrovirus RNA chains and packaging into virus particles in formation of virus particles. For example, the packaging signal sequence is a region from the 3' side of a major splice donor (SD) site to a gag start codon or a region starting from the 3' side of the SD site and containing a part of gag.

As used herein, the term "splice acceptor (SA) sequence" refers to a splice site that is a boundary site between an intron and an exon and is present on the 3' end side of the intron, in RNA processing reaction in which an intron present in RNA is removed and an exon preceding the intron and an exon following the intron are recombined. For example, introns in an RNA precursor present in a nucleus have a consensus sequence of AG at the 3' end, and the 3' end side of the AG sequence is the SA sequence. Conversely, the term "splice donor (SD) sequence" means a splice site present at the 5' end of an intron. For example, introns in an RNA precursor present in a nucleus have a consensus sequence of GU at the 5' end, and the 5' end side of the GU sequence is the SD sequence. The characteristic of the intron consensus sequences for the SA sequence and SD sequence in the eukaryotic mRNA precursor is called a GU-AG rule. The SA sequence and the SD sequence as used herein include an SA sequence and an SD sequence into which a mutation(s) has been introduced into the consensus sequences as long as they function in the RNA processing reaction.

As used herein, the term "gene of interest" means a foreign gene desired to be inserted artificially (by artificial manipulation) into a cell (e.g., a nuclear genome or cytoplasm of a cell) temporarily or permanently. Such a gene includes a gene derived from a cell completely or partially heterologous to a cell into which the gene is introduced, and a gene comprising any mutation. The gene of interest may be the same as an endogenous gene which is naturally occurring in the cell. Au used herein, the term "naturally" means a natural state to which no artificial manipulation has been applied.

As used herein, the term "posttranscriptional regulatory element (PRE)" refers to a sequence that contributes the promotion of polyadenylation of mRNA transcribed from a gene in cells, the promotion of nuclear export of mRNA, or the activation of mRNA translation. The expression of a gene of interest is enhanced at the protein level by inserting the PRE into the untranslated region of the gene of interest contained in the nucleic acid construct of the present invention.

As used herein, the term "wild type" means a gene or a gene product that is isolated from a naturally occurring source and is most frequently found in a population. The wild type gene or gene product may be isolated from nature or artificially made. On the other hand, the term "mutant" refers to a gene or a gene product having an altered sequence and/or altered functional property as compared to the wild-type gene or gene product. A mutant gene is produced by spontaneous mutation or by artificially modifying a gene to mutate its sequence.

As used herein, the term "T cell", also referred to as a T lymphocyte, means a cell derived from a thymus among lymphocytes involved in immune response. The T cell includes a helper T cell, a suppressor T cell, a regulatory T cell, CTL, a naive T cell, a memory T cell, an αβ T cell that expresses α-chain and β-chain TCR, and a γδ T cell that expresses γ-chain and δ-chain TCR. The "cell capable of differentiating into a T cell" is not particularly limited as long as the cell can differentiate into a T cell in vivo or by artificial stimulation. Examples of such a cell include, but not limited to, a hematopoietic stem cell, a pluripotent progenitor cell, a progenitor cell common to the lymph system, and a T cell progenitor cell. Examples of the "cell population containing a T cell or a cell capable of differentiating into a T cell" include blood (peripheral blood, umbilical cord blood, etc.), bone marrow fluid, and a cell population containing a peripheral-blood mononuclear cell (PBMC), a blood cell, a hematopoietic stem cell, a umbilical cord blood mononuclear cell, etc. collected, isolated, purified, or induced from the blood or bone marrow fluid. Various cell populations containing T cells which are derived from blood cells can be used in the present invention. These cells may be activated in vivo or ex vivo by an anti-CD3 antibody or cytokines such as IL-2. These cells may be collected from a living body or obtained by culturing ex vivo. For example, a T cell population obtained from a living body may be used as it is or after cryopreservation.

As used herein, the term "suppression of expression" means suppressing the final polypeptide production, i.e., decreasing the amount of the polypeptide as a product, by inhibiting transcription and/or translation of a gene encoding the polypeptide. Therefore, the "suppression of expression" includes suppression of protein production as a result of rapid degradation of a transcript (mRNA) even when the transcription reaction of a gene encoding the polypeptide is not suppressed. The suppression of expression includes a decrease of the expression level by 20% or more, 40% or more, 60% or more, or 80% or more, and a decrease of the expression level by 100%, that is, complete suppression, as compared to a case where the expression is not suppressed.

Hereinafter, the present invention is specifically described.

### (1) Nucleic acid construct of the present invention

The nucleic acid construct of the present invention is a nucleic acid construct for expressing a gene of interest, comprising: in order from the 5' end to 3' end,
(a) a 5' LTR (Long Terminal Repeat) sequence derived from a retrovirus,
(b) a packaging signal sequence (ψ) derived from a retrovirus,
(c) a sequence of the gene of interest or a multiple cloning site,
(d) a posttranscriptional regulatory element (PRE),
(e) a siRNA-producing sequence which is transcribed into an RNA that forms at least one stem-loop structure and induces RNA interference in a mammalian cell, and
(f) a 3' LTR sequence derived from a retrovirus.
The present invention enables the suppression of expression of a specific endogenous gene in a cell and the expression of a gene of interest introduced into the cell by introducing at least one nucleic acid construct into the cell. The nucleic acid construct of the present invention has high siRNA production efficiency and/or high gene of interest-expression efficiency. Therefore, the nucleic acid construct of the present invention can be used for the purpose of replacing the function of a gene expressed in a cell with another gene newly introduced into the cell, for example, for the purpose of suppressing the expression of an endogenous mutant gene while expressing an introduced wild-type gene.

The nucleic acid constructs of the present invention can be used to produce a retroviral vector. The retroviral vector of the present invention containing a transcript from the nucleic acid construct of the present invention can be produced by introducing the nucleic acid construct of the present invention into a cell capable of producing retroviral particles. Retrovirus is a single-stranded RNA virus, and the viral genome comprises, as major elements, a 5' LTR sequence, an SD sequence, a packaging signal sequence, a gag gene, a pol gene, an SA sequence, an env gene, and a 3' LTR sequence in order from the 5' end to 3' end. Lentivirus as described later comprises a plurality of accessory genes in addition to these elements. Of these, the 5' LTR sequence, the packaging signal sequence and the 3' LTR sequence are essential for retroviral vectors in gene transfer systems using retroviral vectors. The nucleic acid of the present invention comprises all of the essential elements. Gene products of other elements including gag, pol, and env may be supplied from packaging cells carrying these genes. The gene of interest is usually located on the 3' side of the packaging signal sequence in a retroviral vector, or on the 3' side of the packaging signal sequence and the SA sequence when the retroviral vector has the SA sequence.

The (a) 5' LTR sequence, (f) 3' LTR sequence and (b) packaging signal sequence contained in the nucleic acid construct of the present invention may be any sequences as long as they are derived from retrovirus and capable of producing a retrovirus that contains an RNA having these sequences as a genome. Retrovirus includes subclasses of oncoretrovirus and lentivirus. Sequences derived from either subclass of retrovirus can be used in the present invention. These sequences may be derived from the same virus, or may be derived from different viruses to the extent that virus particles can be formed and these sequences can be integrated into the introduced cell's genome by combining the sequences with an appropriate packaging cell.

Examples of the LTR sequences and packaging signal sequence used in the present invention include sequences derived from Moloney murine leukemia virus (MMLV), murine embryonic stem cell virus (MESV), murine stem cell virus (MSCV), myeloid proliferative sarcoma virus (MPSV), and splenic focus-forming virus (SFFV), which belong to oncoretrovirus. Though viral vectors derived from oncoretrovirus enable gene introduction with high efficiency, active cell division of cells is required when the vectors are introduced into the cells. The oncoretroviral vectors have been explained in numerous literature [e.g., US Patent No. 5,219,740, US Patent No. 6,207,453, US Patent No. 5,219,740, BioTechniques, Vol. 7, pp. 980-990 (1989), Human Gene Therapy, Vol. 1, pp. 5-14 (1990), Virology, Vol. 180, 849-852 (1991), Proc. Natl. Acad. Sci. USA, Vol. 90, pp. 8033-8037 (1993), and Cur. Opin. Genet. Develop., Vol. 3, pp. 102-109 (1993)].

Examples of the LTR sequences and packaging signal sequence used in the present invention also include sequences derived from human immunodeficiency virus (HIV-1, HIV-2), monkey immunodeficiency virus (SIV), feline immunodeficiency virus (FIV), equine infectious anemia virus (EIAV), and caprine arthritis encephalitis virus (CAEV), which belong to lentivirus. Lentiviral vectors enable gene introduction into genomes in nuclei regardless of the mitosis of cells into which the gene is introduced. The lentiviral vectors have also been explained in numerous literature [e.g., J. Virology, Vol. 72, pp. 846-8471 (1998)]. Groups of other retroviruses such as the genus Spumavirus (e.g., foamy virus) also enable efficient gene introduction into non-dividing cells.

As the LTR sequences used in the present invention, LTR sequences comprising a mutation(s) can also be used. The LTR is functionally divided into three regions: U3, R, and U5 from the 5' end. The U3 region has enhancer/promoter activity. The viral genome from the R region of the 5' LTR sequence to the R region of the 3' LTR sequence is transcribed by RNA polymerase II of a host cell. LTR sequences in which the U3 region of the 3' LTR sequence and/or the 5' LTR sequence is replaced with an enhancer/promoter derived from a virus other than a virus from which the LTR sequences are derived can also be used in the present invention. Such an exogenous enhancer/promoter that replaces the U3 region may be a virusor mammal-derived sequence, and may be a constitutive, inducible, or tissue-specific enhancer/promoter. Examples of the exogenous enhancer/promoter include enhancers/promoters derived from virus such as human cytomegalovirus (HCMV) immediate early, Moloney murine sarcoma virus (MMSV), murine stem cell virus (MSCV), Rous sarcoma virus (RSV), and spleen focus-forming virus (SFFV), and mammal-derived enhancers/promoters of actin, globin, elastase, albumin, α-fetoprotein and insulin gene.

As the LTR sequences used in the present invention, a 5' LTR sequence and/or a 3' LTR sequence in which a mutation is introduced into the U3 region to lose the enhancer/promoter activity can be used. A mutation is introduced into the U3 region of the 3' LTR sequence contained in the nucleic acid construct for producing a retrovirus vector to lose the enhancer/promoter activity, and thereby transcription from the R region in a provirus is suppressed, wherein the provirus is formed by integration of the viral genome containing a transcript from the nucleic acid construct into chromosomes. A retroviral vector thus obtained in which the U3 region of the 3' LTR sequence is mutated is called a self-inactivating (SIN) vector. The introduction of a mutation into the 3' LTR sequence is carried out by base substitution or base deletion. When the SIN vector is used, since transcription from the R region of the LTR in a provirus is suppressed, it is necessary to arrange a promoter sequence for expressing a gene of interest separately from the LTR. Furthermore, for expressing two or more genes of interest, a promoter sequence different from that of the LTR is arranged. Such a promoter sequence existing between the 5' LTR and the 3' LTR is also referred to as an internal promoter sequence. As the internal promoter sequence, a virus-derived promoter sequence or a mammalian gene-derived promotor sequence can be used. The virus-derived promoter sequence can be a sequence derived from the same virus as or a different virus from a virus from which the 5' LTR, 3' LTR or packaging signal sequence is derived. Examples of the virus-derived promoter sequence include a promoter sequence derived from the U3 region of the LTR of a retrovirus, and a promoter sequence derived from the U3 region of the LTR of a murine stem cell virus (MSCV). Further, examples of the internal promoter sequence include the sequences cited as examples of the exogenous promoter that replaces the U3 region of the 5' LTR sequence as described in the previous paragraph. Examples of the virus-derived promoter sequence further include an SV40 promoter sequence and a CMV promoter sequence. Furthermore, a sequence of a promoter that functions in a mammalian cell, such as PGK promoter, EF1-α promoter, β-actin promoter, CAG promoter or the like can also be used. The internal promoter may be placed upstream of (c), and may be placed between (a) and (c), preferably between (b) and (c).

The nucleic acid construct of the present invention may comprise an SD sequence and/or an SA sequence. The SD sequence and/or SA sequence may be an SD sequence and/or an SA sequence contained in an mRNA transcribed from a promoter used, or an SD sequence and/or an SA sequence foreign to the promoter sequence used, that is, an SD sequence and/or an SA sequence derived from a different gene from the promoter sequence used. The phrase "derived from a different gene" means that the above-mentioned elements are derived from different genes of the same virus or mammal or derived from different viruses or mammals. Further, SD and/or SA sequences derived from a virus that is the same as or different from a virus from which the 5' LTR, 3' LTR or packaging signal sequence is derived, or derived from a mammalian gene can be used. The SD sequence and the SA sequence may be derived from different genes from each other. For example, an SD sequence and an SA sequence derived from 16S RNA of simian virus (SV) 40, immediate early RNA of HCMV, and human hEF1α gene can be used [Proc. Natl. Acad. Sci. USA, vol. 95, No. 1, pp. 219-223 (1998)]. An SD sequence or an SA sequence in which a mutation is introduced into the consensus sequence to enhance or suppress the splicing activity can also be used for the nucleic acid construct of the present invention.

The (e) siRNA-producing sequence contained in the nucleic acid construct of the present invention is a sequence to be transcribed into an RNA that forms at least one stem-loop structure and induces RNA interference in a mammalian cell. The RNA interference as used herein is intended to selectively suppress the expression of a specific endogenous gene being naturally expressed in a cell into which the vector is introduced. The RNA interference is induced by an siRNA formed by annealing an RNA molecule homologous to a nucleotide sequence of an mRNA transcribed from a gene whose expression is desired to be suppressed (hereinafter referred to as a target gene) and an RNA molecule complementary to the nucleotide sequence.

In the siRNA-producing sequence used in the present invention, a homologous sequence (sense sequence) and a complementary sequence (antisense sequence) to a nucleotide sequence of a region of an mRNA transcribed from a target gene are arranged in tandem. A single RNA strand transcribed from the siRNA-producing sequence forms a double-stranded structure by annealing of the sense sequence and the antisense sequence in the molecule, and therefore, forms a stem-loop structure of a stem region that is the formed double-stranded RNA portion and a loop region that is any sequence arranged between the antisense sequence and the antisense sequence. In a cell, the siRNA is produced from the stem region by the action of RNase III (Dicer). A portion in the siRNA-producing sequence corresponding to the stem region has a length of, for example, 13 to 29 nucleotides, preferably 15 to 25 nucleotides, and more preferably 19 to 25 nucleotides from the viewpoint of interferon response suppression in mammalian cells. The loop region may have any sequence. For example, the loop region may be a sequence having a length of 1 to 30 nucleotides, preferably 1 to 25 nucleotides, and more preferably 5 to 22 nucleotides.

The siRNA produced in cells according to the present invention is composed of an RNA having a sequence homologous to a specific nucleotide sequence of an mRNA transcribed from a target gene and an RNA having a sequence complementary to the specific nucleotide sequence. Each RNA does not need to be completely homologous or complementary to the specific nucleotide sequence. To the extent that the function of suppressing the expression of a target gene is exerted, a sequence for producing a siRNA consisting of an RNA having a substantially homologous sequence and an RNA having a substantially complementary sequence may be used. The siRNA-producing sequence used in the present invention may be a sequence transcribed into one type of siRNA targeting one type of gene, a sequence transcribed into a plurality of siRNAs corresponding to nucleotide sequences of different regions from one type of target gene, or a sequence transcribed into a plurality of siRNAs corresponding to a plurality of target genes. For example, the siRNA-producing sequence used in the present invention produces 1 to 10, 1 to 6, 1 to 4, plural or several siRNAs.

As one aspect of the present invention, transcription from the (e) siRNA-producing sequence into an siRNA is accomplished by use of a promoter sequence that controls the expression of the (c) sequence of the gene of interest. In other words, there is not a promotor sequence between (c) and (e) and an RNA to be transcribed from the sequence of a gene of interest and an siRNA are transcribed as a series of RNA molecules. As one aspect of the present invention, depending on the property of the promoter sequence as described above and the required amount of siRNA transcription, a new promoter sequence can be arranged between (c) and (e).

The (c) sequence of the gene of interest contained in the vector of the present invention is a gene sequence desired to be expressed in a cell into which the vector is introduced. Examples of the gene sequence include a protein-encoding sequence, and a sequence encoding an RNA that functions in a cell such as tRNA or miRNA. The vector of the present invention may contain a sequence in which a plurality of restriction enzyme recognition sequences for linking a sequence of a gene of interest are arranged (multiple cloning site) as the sequence (c), and then the sequence of the gene of interest may be inserted into the vector by utilizing the multiple cloning site. Such a vector that contains a multiple cloning site instead of a sequence of a gene of interest is also included in the vector of the present invention.

The siRNA generated from the nucleic acid sequence of (e) contained in the vector of the present invention may target the gene sequence of (c) and thereby suppress the gene expression from the gene sequence. In a case where such a phenomenon is expected, in order to prevent the phenomenon, a mutation can be introduced into the gene sequence of (c) to modify the gene sequence so as not to be affected by the siRNA.

It is known that there are 1 to 6 types of codons (combinations of three bases) that specify each type of amino acid on a gene. Appropriate codon selection enables base substitution without altering the encoded amino acid sequence (called a silent mutation). The Silent mutation is often attained by converting the third base of a codon. When the gene of interest has the same nucleotide sequence as a region on the target gene corresponding to the siRNA generated from the sequence of (e), the siRNA suppresses the expression of both the original target gene and the gene of interest introduced. When the silent mutation is introduced into the gene of interest to eliminate the homology/complementarity with the siRNA sequence, the siRNA does not act on the RNA transcribed from the gene of interest, and therefore its expression suppression is reduced. Thus the expression of an endogenous gene can be selectively suppressed in a region where the siRNA acts, even when the amino acid sequence of the polypeptide encoded by the gene of interest is the same as the amino acid sequence of the polypeptide encoded by the endogenous gene that is the target of the siRNA.

Further, as another embodiment of the present invention wherein the nucleotide sequence of a gene of interest is mutated, the amino acid sequence encoded by the RNA on which the siRNA acts may be altered by replacement with other amino acids, for example replacement with similar amino acids, as long as the function of the polypeptide encoded by the gene of interest is not impaired. The similar amino acids mean amino acids similar in physicochemical properties. Examples of the similar amino acids include amino acids classified into the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids with hydroxyl groups (Ser, Thr), or amino acids with small side chains (Gly, Ala, Ser, Thr, Met). Such replacement with similar amino acids is expected to bring about no change in the phenotype of a polypeptide (i.e., conservative amino acid replacement). Specific examples of conservative amino acid replacement are well known in the art and are described in various literature [see, e.g., Bowie et al., Science, Vol. 247, pp. 1306-1310 (1990)]. When the conservative amino acid replacement mutation is introduced into a gene of interest, the siRNA produced from the sequence of (e) does not act on the RNA transcribed from the gene of interest, thereby the suppression of expression is reduced. Thus the expression of an endogenous polypeptide can be selectively suppressed in a region where the siRNA acts, even when the amino acid sequence of the polypeptide encoded by the gene of interest is similar to the amino acid sequence of the endogenous polypeptide.

Hereinafter, a gene into which the silent mutation or the conservative amino acid replacement mutation has been introduced as described above may be referred to as a "codon-converted" gene. For example, the expression efficiency of a gene of interest is expected to be improved by selecting codons frequently used in a host to be used or a sequence that increases the translation efficiency to convert the nucleotide sequence as described above.

As one aspect of the present invention, the sequence of a gene of interest contained in the vector of the present invention is a sequence encoding an oligomer protein. The oligomer protein includes a structural protein, an enzyme, a transcription factor, a receptor, and an antibody. Further, in the present invention, the oligomer protein may be a cell surface protein (membrane protein). The sequence of a gene of interest is preferably a sequence encoding an antigen recognition receptor, for example a sequence encoding a T cell receptor (T cell receptor: TCR), as described in Examples.

There are two types of TCRs: a heterodimer consisting of an α chain and a β chain and a heterodimer consisting of a γ chain and a δ chain. Each chain of TCR consists of a variable (V) region, a joining (J) region, a constant (C) region, and the like. The diversity of the V region of TCR results from combinations of gene segments encoding the V region due to DNA rearrangement and shifts of rearrangement joining sites, and insertion of an N sequence into the joining sites. In the V regions of the α chain and β chain, a hypervariable region (CDR) comprising particularly high amino acid sequence variation is found. As one aspect of the present invention, the gene of interest may be a sequence in which two genes encoding two polypeptides constituting a heterodimer of TCR are ligated in a polycistronic manner. The two genes can be interconnected via a sequence selected from the group consisting of a sequence encoding a self-cleaving peptide and an IRES (internal ribosome entry site) sequence. The order of the two genes to be connected is not limited, and for example, they may be a polycistronic sequence of, in order from the 5' end to 3' end, TCR α chain-TCR β chain or TCR β chain-TCR α chain.

The self-cleaving peptide can be obtained from a viral 2A peptide or a 2A-like peptides having function equivalent to the viral 2A peptide. For example, the self-cleaving peptide can be selected from the group consisting of a 2A peptide (F2A) derived from foot-and-mouth disease virus (FMDV), a 2A peptide (E2A) derived from equine rhinitis A virus (ERAV), a 2A peptide (P2A) derived from Porcine teschovirus (PTV-1), and a 2A peptides (T2A) derived from Thosea asigna virus (TaV). For example, P2A having an amino acid sequence encoded by a nucleotide sequence set forth in SEQ ID NO: 17 can be used. The 2A peptide is reviewed in Expert Opin Biol Ther, Vol. 5, pp. 627-638 (2005).

The IRES sequence refers to an element that facilitates the direct entry of ribosomes into the initiation codons of cistrons (protein-coding regions), e.g. AUG and thereby initiates cap-independent translation of the genes [Trends Biochem. Sci., Vol. 15, No. 12, pp. 477-83 (1990)]. Two or more polypeptides are translated from a single mRNA having two or more cistrons linked via IRES sequences.

For example, when an exogenous TCR that recognizes a desired antigen is introduced into a T cell, the exogenous TCR can compete with the endogenous TCR naturally expressed in the T cell and thereby the expression of the exogenous TCR can be decreased. In addition, mispairing of the exogenous TCR and the endogenous TCR can occur, and thereby side effects such as graft-versus-host disease (GVHD) can be produced. Therefore, it is important to suppress the expression of endogenous TCRs. The V regions of the endogenous TCRs differ depending on individual TCRs, while the C regions have a sequence common to TCRs and are encoded by genes having the same nucleotide sequence. Therefore, the C region is a suitable target of the siRNA produced from the sequence of (e). Furthermore, in order to prevent the siRNA from suppressing the expression of the exogenous TCR gene introduced, the silent mutation can be introduced into the nucleotide sequence encoding the C region in the gene. Thus, the exogenous TCR can be efficiently expressed.

For example, for suppressing the expression of endogenous TCRs, a nucleotide sequence of the exogenous TCR α chain corresponding to a nucleotide sequence of the endogenous TCR α chain: AGTAAGGATTCTGATGTGTAT (SEQ ID NO: 1) in the C region of the TCR-encoding gene may be changed to AGCAAGGACAGCGACGTGTAC (SEQ ID NO: 2) by codon conversion. Both the above-mentioned two nucleotide sequences encode the amino acid sequence: SKDSDVY (SEQ ID NO: 3). However, the α chain of the endogenous TCR is selectively suppressed by the siRNA produced by transcription of an artificial gene set forth in SEQ ID NO: 13. Examples of the codon conversion are shown in Table 1 in Examples.

The sequence for producing an siRNA that suppresses the expression of endogenous TCRs can also be combined with a "sequence encoding a chimeric antigen receptor as a gene of interest" described later. The nucleic acid construct comprising such a combination is useful for preparation of T cells that have lost the cytotoxic activity derived from TCRs and exert an action faithful to the specificity of CARs.

As one aspect of the present invention, the sequence of a gene of interest contained in the nucleic acid construct of the present invention is a sequence encoding a chimeric antigen receptor (CAR). A typical CAR structure is composed of a single chain antibody (single chain variable fragment: scFv) that recognizes a surface antigen of a tumor cell, a transmembrane domain, and an intracellular domain that activates T cells. As the intracellular domain, the intracellular domain of TCR complex CD3ζ is preferably used. CARs having such a configuration are called first generation CARs. The gene for the single chain antibody portion is isolated from, for example, a hybridoma producing a monoclonal antibody that recognizes a target antigen. CAR-expressing T cells directly recognize the surface antigens of tumor cells independently of the expression of major histocompatibility antigen class I on the tumor cells, and at the same time, activate the T cells, and thereby can efficiently kill the tumor cells.

For the purpose of enhancing the ability of first generation CARs to activate T cells, second generation CARs in which an intracellular domain of a T cell costimulatory molecule is linked have been developed. Examples of the T cell costimulatory molecule include CD28, and CD137 (4-1BB) and CD134 (OX40) which are tumor necrosis factor (TNF) receptor superfamily. As a further improvement, third-generation CARs in which intracellular domains of these costimulatory molecules are linked in tandem have also been developed. Many CAR molecules targeting various tumor antigens have been reported. The nucleic acid construct of the present invention may comprise a sequence encoding any CAR as the sequence of a gene of interest.

As one aspect of the present invention, the siRNA produced by a nucleic acid contained in the nucleic acid construct of the present invention acts on an mRNA encoding a molecule that suppresses the activity of immune cells (particularly T cells) and suppresses its expression. In other words, the siRNA targets a gene sequence encoding a molecule that suppresses the activity of immune cells. As the molecule that suppresses the activity of immune cells, NR4A1, NR4A3, TGFBR2, and TET2 are known in addition to an immune checkpoint molecule. Examples of the immune checkpoint molecule include, but not limited to, PD-1 (CD279, GenBank accession number: M_005018), CTLA-4 (CD152, GenBank accession number AF414120.1), LAG 3 (CD223, GenBank accession number: NM_002286.5), Tim3 (HAVCR2, GenBank accession number: JX049979.1), BTLA (CD272, GenBank accession number: NM_181780.3), BY55 (CD160, GenBank accession number: CR5418888.1), TIGIT (VSTM3, GenBank accession number: NM_173799), B7H5 (C10orf54, GenBank accession number: NM_022153.1), LAIR1 (CD305, GenBank accession number: CR542051.1), SIGLEC10 (GenBank accession number: AY358337.1), and 2B4 (CD244, GeneBank accession number: NM_0011666664.1). For example, CTLA-4 is a cell surface protein expressed on specific CD4 and CD8 T cells. When CTLA-4 is bound to its ligands (B7-1 and B7-2) on antigen-presenting cells, T cell activation and effector function are inhibited. Therefore, the vector of the present invention can suppress the suppression of T cell activity, that is, can activate T cells. As one aspect of the present invention, the cell into which the nucleic acid construct is introduced is an immune cell including a T cell. When the sequence of a gene of interest is a CAR-encoding sequence, it is preferable to suppress the expression of a molecule that suppresses the activity of immune cells.

The sequence for producing an siRNA that suppresses the expression of a molecule that suppresses the activity of T cells can also be combined with the above-described "sequence encoding TCR as a gene of interest". The nucleic acid construct comprising such a combination is useful for preparation of TCR-expressing T cells that recognize specific antigens and can maintain the activity in vivo for an extended period of time.

The (d) posttranscriptional regulatory element (PRE) contained in the nucleic acid construct of the present invention is useful for enhancing the expression of a gene of interest. The PRE is placed within introns of a transcript from the nucleic acid construct, and may be removed by splicing in the life cycle of retrovirus. Examples of the PRE that is not removed by splicing include a posttranscriptional processing element of herpes simplex virus, and posttranscriptional regulatory elements of hepatitis B virus (HPRE) and Woodchuck hepatitis virus (WPRE). Further, a mutant of PRE, for example, a mutant in which the expression of X protein is suppressed can also be used in the present invention. In the present invention, WPRE is preferably used. Regarding WPRE, see US Patent No. 6,136,597 or US Patent No. 7,419,829.

The nucleic acid construct of the present invention may comprise a Rev response element (RRE) sequence and/or a central polypurine tract (cPPT) sequence. Examples of the RRE include, but not limited to, an RRE located at position 7622-8459 of HIV NL4-3 genome (GenBank accession number: AF003887), and RREs derived from other strains of HIV or other retroviruses. The cPPT means a sequence of about 15 bases existing roughly in the center of the lentivirus genome, and acts as a primer binding site for synthesis of a plus-stranded DNA in the process of synthesizing a double-stranded DNA from a lentivirus genomic RNA. When the lentivirus RNA genome is reverse transcribed, the cPPT functions together with a central termination sequence (CTS) to form a triple-stranded structure called a DNA flap. The nucleic acid construct of the present invention may comprise cPPT and RRE or RRE and cPPT in order from the 5' end to the 3' end.

As one aspect of the present invention, the vector of the present invention suitably contains a 5' LTR sequence, a packaging signal sequence, a cPPT sequence, an RRE sequence, an internal promoter sequence, an SD sequence, an SA sequence, a sequence of a gene of interest, a PRE sequence, a siRNA-producing sequence, and a 3' LTR sequence, in the order from 5' to 3'.

In the method of the present invention, an appropriate packaging cell can be used to prepare a retroviral vector particle, wherein the appropriate packaging cell is selected as the cell capable of producing a retrovirus particle, depending on the LTR sequence and the packaging signal sequence possessed by the nucleic acid construct. The retroviral vector particle contains a transcript from the nucleic acid construct of the present invention. Examples of the packaging cell include PG13 (ATCC CRL-10686), and PA317 (ATCC CRL-9878). The retrovirus particle can also be prepared by introducing necessary components (gag gene, pol gene, envelope gene and other accessory genes) for production of retrovirus particles into a 293 cell or 293 T cell having high transfection efficiency, and then using the cell as a host (as the cell capable of producing a retrovirus particle). Packaging cells that can be used for packaging retroviral vectors and plasmid kits for producing retroviruses are widely commercially available from many companies. These commercially available packaging cells and kits can be used in the method of the present invention.

In the present invention, a retrovirus produced by pseudotyped packaging, which has an envelope derived from a virus heterologous to a virus from which the genome of the retroviral vector is derived, can be also used. For example, a pseudotyped retrovirus having an envelope derived from MMLV, gibbon ape leukemia virus (GaLV), vesicular stomatitis virus (VSV), or feline endogenous virus, or a protein that can function as the envelope can be used. Furthermore, the retroviral vector particle having a glycosylated protein on the surface can be prepared by using a packaging cell into which an enzyme gene or the like involved in glycosylation has been introduced.

After culturing the retrovirus-producing cell prepared in such a manner as described above, the retrovirus particle can be obtained by centrifuging the cell culture to collect a supernatant and then removing contaminants by appropriate filtration. Though the crudely purified retrovirus particle thus obtained may be directly contacted with a cell to perform gene introduction, the retrovirus particle may be purified by a known method to prepare a retrovirus particle having higher purity and the retrovirus particle thus obtained may be used for gene introduction.

The present invention provides a composition comprising the retroviral vector of the present invention as an active ingredient together with a pharmaceutically acceptable excipient. Suitable pharmaceutically acceptable excipients are well known to those of skill in the art. Examples of the pharmaceutically acceptable excipient include a phosphate buffered saline (e.g. 0.01 M phosphate, 0.138 M NaCl, 0.0027 M KCl, PH 7.4), an aqueous solution containing a mineral salt such as hydrochloride, hydrobromide, phosphate, or sulfate, a physiological saline solution, a solution such as glycol or ethanol, and salts of organic acid such as acetate, propionate, malonate, and benzoate. An auxiliary agent such as a wetting agent or an emulsifier, and a pH buffering agent can also be used. Pharmaceutically acceptable excipients described in Remington's Pharmaceutical Sciences (Mack Pub. Co., N. J. 1991) (which is incorporated herein by reference) can be used as appropriate. The composition can take a known form suitable for parenteral administration, e.g. injection or infusion. In addition, pharmaceutical additives such as a suspending agent, a preservative, a stabilizer and/or a dispersant, and a preservative for extending the shelf life during storage can be used. The composition may be in a dry form for reconstitution with a suitable sterile liquid prior to use.

As one aspect of the present invention, the nucleic acid construct of the present invention is a nucleic acid construct for expressing a gene of interest, and comprises, in order from the 5' end to the 3' end,
(c) a sequence of the gene of interest,
(d) a posttranscriptional regulatory element (PRE),
(e) a siRNA-producing sequence which is transcribed into an RNA that forms at least one stem-loop structure and induces RNA interference in a mammalian cell. A cell containing this nucleic acid construct is also included in the present invention. In the cell, the expression of a specific endogenous gene is suppressed and the gene of interest is expressed. The nucleic acid construct may be introduced into a cell with a suitable vector such as a plasmid vector, a viral vector other than a retrovirus, or a transposon vector so that the effect can be stably exerted in the cell. In addition, the nucleic acid construct may be integrated onto the chromosomal DNA of a cell using a genome editing technique. This nucleic acid construct may be also introduced directly into a cell. To introduce the nucleic acid construct into a cell directly or with a plasmid vector, a method comprising use of a carrier such as a liposome or a ligand-polylysine, a calcium phosphate method, an electroporation method, a particle gun method, or the like can be used. The viral vector is not particularly limited, and a known viral vector usually used for gene introduction is used. Examples of the known viral vector include an adenovirus vector, an adeno-associated virus vector, a Simian virus vector, a vaccinia virus vector, a measles virus vector, and a Sendai virus vector.

### (2) Method for producing gene-introduced cell of the present invention

The method for producing a gene-introduced cell of the present invention is characterized by comprising a step of introducing a retroviral vector containing the nucleic acid construct of the present invention as described above in (1) or a transcript from the nucleic acid construct into a cell. As one aspect of the present invention, the step is performed ex vivo.

In the method of the present invention, mammalian cells, for example, cells derived from human or non-human mammals such as monkeys, mice, rats, pigs, cows, dogs and the like can be used. The cells that can be used in the method of the present invention are not particularly limited, and any cell can be used. For example, cells collected, isolated, purified, or induced from blood (peripheral blood, umbilical cord blood, etc.), body fluids such as bone marrow, tissues or organs can be used. Peripheral blood mononuclear cells (PBMC), immune cells [T cells, dendritic cells, B cells, hematopoietic stem cells, macrophages, monocytes, NK cells or blood cells (neutrophils, basophile)], umbilical cord blood mononuclear cells, fibroblasts, preadipocytes, hepatocytes, cutaneous keratinocytes, mesenchymal stem cells, adipose stem cells, various cancer cell lines or neural stem cells can be used. In the present invention, T cells, T cell progenitor cells (hematopoietic stem cells, lymphocyte progenitor cells, etc.), or cell populations containing T cells or T cell progenitor cells are preferably used. The T cells include αβ T cells, γδ T cells, CD8 positive T cells, CD4 positive T cells, regulatory T cells, cytotoxic T cells, and tumor-infiltrating lymphocytes. The cell populations containing T cells or T cell progenitor cells include PBMCs. The above-described cells may be collected from living bodies, may be obtained by expansion of a cell collected from a living body, or may be established cell lines. When the produced cell or a cell differentiated from the produced cell is transplanted into a living body, it is preferable that the cell is derived from a cell collected from the living body itself or a homogenous living body.

In the step of introducing the retroviral vector of the present invention into a cell, a functional substance that improves introduction efficiency can also be used (for example, WO95/26200, WO00/01836). Examples of the substance that improves introduction efficiency include a substance having an activity of binding to a viral vector, for example, fibronectin or a fibronectin fragment. A fibronectin fragment having a heparin binding site, for example, a commercially available fragment such as RetroNectin (registered trademark, CH-296, manufactured by Takara Bio Inc.) can be preferably used.

In a preferred aspect of the present invention, the functional substance can be used in a state of being immobilized on a suitable solid phase, for example, a container (plate, petri dish, flask or bag, etc.) or carrier (microbeads, etc.) used for cell culture.

### (3) Cell of the present invention

The cell of the present invention is a cell containing the nucleic acid construct as described above in (1). For example, the production method as described above in (2) can be used to prepare a cell into which the nucleic acid construct as described above in (1) has been introduced. In the cell of the present invention, an exogenous gene of interest is expressed while the expression of a specific endogenous gene is suppressed.

As one aspect of the present invention, the cell of the present invention can be used as a therapeutic agent for disease. The therapeutic agent contains the cell of the present invention capable of expressing a gene of interest useful for treating a disease as an active ingredient, and may further contain a suitable excipient. The excipient is not particularly limited as long as it is pharmaceutically acceptable. Examples of the excipient include a stabilizer, a buffer, and a tonicity agent. The disease for which the cell of the present invention is administered is not particularly limited as long as the disease is sensitive to the cell. Examples of the disease include cancers [blood cancer (leukemia), solid tumor, etc.], inflammatory diseases /autoimmune diseases (asthma, eczema, etc.), hepatitis, and infectious diseases caused by viruses, bacteria, and fungi (influenza, AIDS, tuberculosis, MRSA infection, VRE infection, and deep mycosis). For the treatment of these diseases, cells expressing TCRs or CARs that recognize antigens possessed by cells whose reduction or loss is desired in the diseases, namely, tumor antigens, viral antigens, bacterial antigens, etc. are administered. The cell of the present invention can also be used for prevention of infectious diseases after bone marrow transplantation or irradiation, donor lymphocyte infusion for the purpose of relieving recurrent leukemia, and the like. The therapeutic agent containing the cell of the present invention as an active ingredient can be administered, for example but not limited to, parenterally, for example, intradermally, intramuscularly, subcutaneously, intraperitoneally, intranasally, intraarterially, intravenously, intratumorally, or into efferent lymph vessels etc., for example, by injection or infusion.

Hereinafter, the present invention is explained in more detail with reference to examples, which the present invention is not limited to.

Among operations described herein, basic operations were performed by methods as described in Molecular Cloning: A Laboratory Manual 3rd ed., edited by T. Maniatis et al., published in 2001 by Cold Spring Harbor Laboratory.

### Example 1: Preparation of codon-converted human T cell receptor α and β genes

According to An J et al., Int. J Hematol., Vol. 93, pp. 176-185 (2011), nucleic acid fragments containing a TCR α-chain gene and a TCR β-chain gene that recognize a peptide of amino acids 235-243 of tumor antigen WT1 were prepared. Then, nucleotide sequences (SEQ ID NOs: 1 and 4) encoding amino acid sequences of a C region shown by SEQ ID NOs: 3 and 6 respectively in the TCR α-chain gene thus obtained were changed to codon-converted nucleotide sequences shown by SEQ ID NOs: 2 and 5, respectively. Similarly, nucleotide sequences (SEQ ID NOs: 7 and 10) encoding amino acid sequences of a C region shown by SEQ ID NOs: 9 and 12 respectively in the TCR β-chain gene were changed to codon-converted nucleotide sequences shown by SEQ ID NOs: 8 and 11, respectively. Details of the above-described nucleotide sequence conversions are shown in Table 1. In addition, an artificial gene shown by SEQ ID NO: 13 was synthesized. The artificial gene is transcribed into four types of single-stranded RNAs that form stem-loop structures. These single-stranded RNAs produce siRNAs that target the nucleotide sequences of SEQ ID NOs: 1, 4, 7, and 10 respectively in cells to suppress the expression of the wild-type TCR α-chain and wild-type TCR β-chain genes. Though the wild-type nucleotide sequence and the codon-converted nucleotide sequence encode the same amino acid sequence, the expression of the codon-converted TCR is not suppressed by the double-stranded siRNA transcribed from the DNA shown by SEQ ID NO: 13 because a mutation has been introduced into the codon-converted TCR nucleotide sequence.

**[Table 1]**

| | Wild-type | Codon converted-type | Amino acid sequence |
|---|---|---|---|
| TCR α | AGTAAGGATTCTGATG TGTAT | AGCAAGGACAGCGACGT GTAC | SKDSDVY |
| | (SEQ ID NO: 1) | (SEQ ID NO: 2) | (SEQ ID NO: 3) |
| | AGCAACAAATCTGACT TTGCA | TCCAACAAGAGCGACTT CGCC | SNKSDFA |
| | (SEQ ID NO: 4) | (SEQ ID NO: 5) | (SEQ ID NO: 6) |
| TCR β | GCCACCATCCTCTATG AGATC | GCCACCATCCTGTACGA GATC | ATILYEI |
| | (SEQ ID NO: 7) | (SEQ ID NO: 8) | (SEQ ID NO: 9) |
| | GCCATGGTCAAGAGAA AGGAT | GCCATGGTGAAGCGGAA GGAC | AMVKRKD |
| | (SEQ ID NO: 10) | (SEQ ID NO: 11) | (SEQ ID NO: 12) |

### Example 2: Preparation of codon-converted TCR-expressing retroviral vector DNA

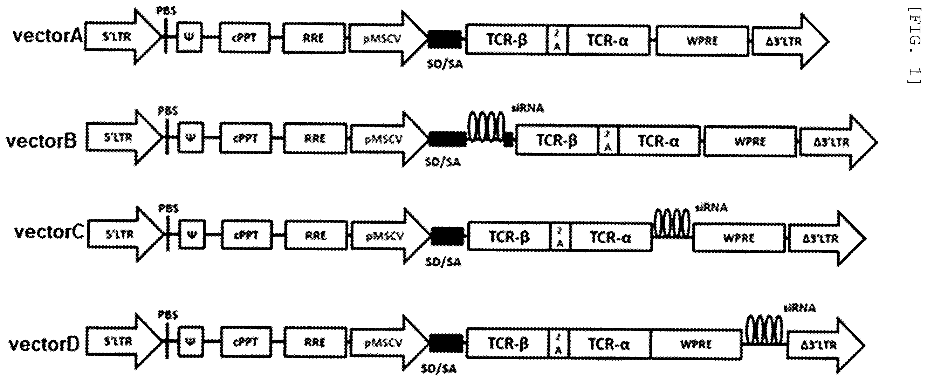

First, PCR was performed using pMSCVneo (manufactured by Clontech) as a template to amplify a DNA fragment of a MSCV U3 promoter site shown by SEQ ID NO: 14. In addition, a genomic DNA was extracted from peripheral blood mononuclear cells (PBMC) isolated from peripheral blood of a human from whom informed consent was obtained, using NucleoSpin (registered trademark) Tissue (manufactured by MACHEREY-NAGEL GmbH & Co.). Using the genomic DNA as a template, PCR was performed with primers shown by SEQ ID NO: 15 and SEQ ID NO: 16 to amplify a DNA fragment of a region containing SD and SA sequences derived from a human EF1α gene. Furthermore, a gene containing a P2A peptide sequence shown by SEQ ID NO: 17 was artificially synthesized. As shown in FIG. 1 (A), the MSCV U3 promoter sequence, the region containing SD and SA sequences derived from the human EF1α gene, and the nucleic acid fragments containing the codon-converted human anti-WTl TCR α-chain gene and the codon-converted human anti-WTl TCR β-chain gene as prepared in Example 1, and the artificial gene encoding the P2A peptide sequence were inserted into a ClaI-MluI digestion product of lentivirus vector DNA, pLVSIN-CMV Neo (manufactured by Takara Bio). The order of a RRE sequence and a cPPT sequence in the lentivirus vector was interchanged, so that vector A comprising the cPPT sequence and the RRE sequence in order from 5' to 3' was prepared. In other words, vector A comprises the 5' LTR, the packaging signal sequence, the cPPT sequence, the RRE sequence, the MSCV LTR U3 promoter sequence, the SD and SA sequences derived from human EF1α gene, the codon-converted WT1-specific TCR α-chain and β-chain gene sequences polycistronically linked via the 2A peptide, and the WPRE sequence, in order from 5' to 3'.

Next, as shown in FIG. 1, the artificial gene for producing four types of siRNAs as synthesized in Example 1 was inserted between the SD and SA sequences of vector A, between the codon-converted WT1-specific TCR gene sequence and the WPRE sequence of vector A, and between the WPRE and the 3' LTR of vector A to prepare vector B, vector C, and vector D, respectively.

### Example 3: Preparation of retrovirus solution

Escherichia coli JM109 was transformed with vectors A to D as prepared in Example 2 to obtain transformants. Plasmid DNAs carried by these transformants were purified using NucleoSpin (registered trademark) Plasmid Midi (manufactured by MACHEREY-NAGEL GmbH & Co.). The purified plasmid DNAs were used as DNAs for transfection in the following experiments.

Using any one of the prepared plasmids of vectors A to D, Lentiviral High Titter Packaging Mix (manufactured by Takara Bio Inc.) and Lenti-X 293T cells (manufactured by Clontech), four types of supernatants containing lentivirus having a VSVG envelope were obtained. The supernatants were filtered through a 0.45 µm filter (Milex HV, manufactured by Millipore) to prepare virus solutions A to D.

### Example 4: Infection of human PBMC with codon-converted TCR and codon-converted TCR-siRNA co-expressing retroviral vector - 1

Peripheral blood mononuclear cells (PBMC) separated from peripheral blood of a human from whom informed consent was obtained were stained with an FITC-labeled anti-human CD8 antibody (manufactured by Becton Dickinson). CD8-positive cells were separated with anti-FITC microbeads (manufactured by Milteny Biotech), and infected with a 2-fold, 6-fold, 18-fold or 54-fold dilution of the virus solutions A to D as prepared in Example 3, using RetroNectin (manufactured by Takara Bio). The cells were collected 7 days after the virus infection, and a total RNA was extracted from the cells and then treated with DNase I using NucleoSpin RNA Plus (manufactured by MACHEREY-NAGEL GmbH & Co.). Using the obtained total RNA as a template, cDNA synthesis was performed using PrimeScript RT reagent Kit (Perfect Real Time) (manufactured by Takara Bio Inc.). Furthermore, using the cDNA as a template, real-time PCR was performed using TB Green Premix Ex Taq II (manufactured by Takara Bio Inc.), and primers for wild-type TCR α-chain gene amplification shown by SEQ ID NOs: 18 and 19, primers for wild-type TCR β-chain gene amplification shown by SEQ ID NOs: 20 and 21, primers for codon-converted TCR α-chain gene amplification shown by SEQ ID NOs: 22 and 23, or primers for codon-converted TCR β-chain gene amplifications shown by SEQ ID NOs: 24 and 25. The expression level of each gene was measured and then calculated as a relative value. The total RNA amount was corrected based on the measured value of a GAPDH gene using primers for GAPDH gene amplification shown by SEQ ID NOs: 26 and 27. Separately, after the cells were collected 7 days after the virus infection and treated with Recombinant DNaseI (RNAse-free) (manufactured by Takara Bio Inc.) to digest plasmid DNAs and the like contained in the virus, a genomic DNA was extracted using NucreoSpin (registered trademark) Tissue (manufactured by MACHEREY-NAGEL GmbH & Co.), and the number of virus copies integrated into the genome was measured using Provirus Copy Number Detection Primer Set, Human (manufactured by Takara Bio Inc.), CycleavePCR Core Kit (manufactured by Takara Bio Inc.) and Lenti-X Provirus Quaqntitation Kit (manufactured by Clontech).

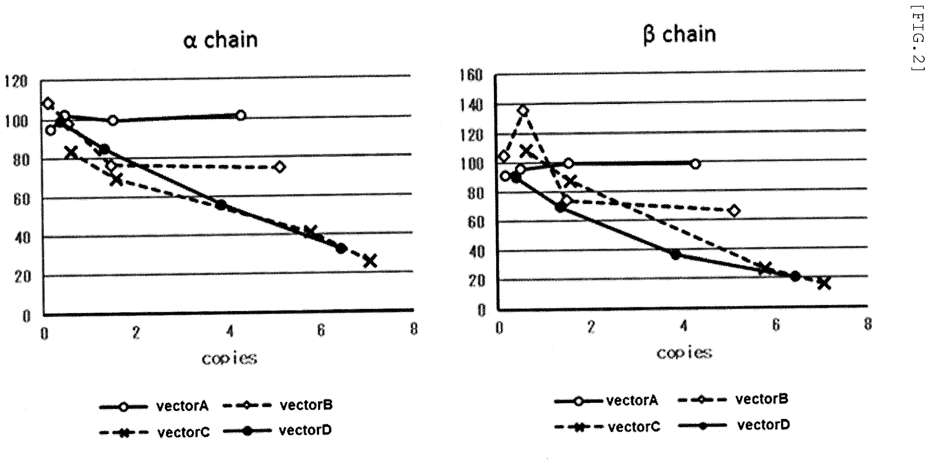

Suppressive effects on the wild-type TCR genes were evaluated by calculating the ratios of values of gene expression in each experimental group relatively to gene expression of the wild-type TCR α-chain and the wild-type TCR β-chain in virus non-infected control cells. Results are shown in FIG. 2. In the figure, the vertical axis shows relative values of gene expression levels when the expression level of the control vector is defined as 100. The horizontal axis shows the number of virus copies. As shown in FIG. 2, vector A did not suppress the expression of the wild-type TCR α-chain and β-chain genes. Vector B weakly suppressed the expression. Vector C and vector D efficiently suppressed the expression. In other words, it was shown that vector C and vector D were excellent in suppressing the expression of endogenous TCR.

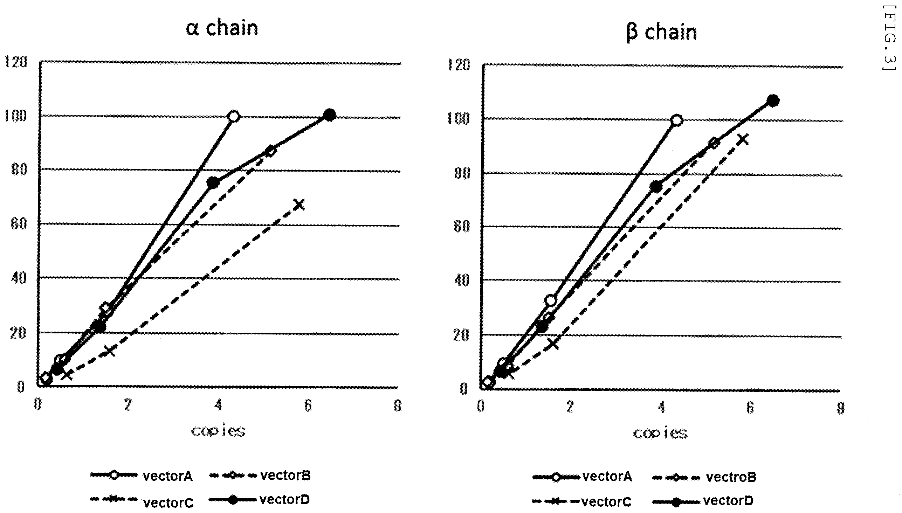

In addition, the expression levels of the codon-converted TCR genes were evaluated by calculating the ratios of values of gene expression in each experimental group relatively to gene expression of the codon-converted TCR α-chain and the codon-converted TCR β-chain in cells infected with the 2-fold diluted virus solution of vector A as a reference. Results are shown in FIG. 3. In the figure, the vertical axis shows relative values of gene expression levels when the expression level of the reference (cells infected with the 2-fold diluted virus solution of vector A) is defined as 100. The horizontal axis shows the number of virus copies. As shown in FIG. 3, the cells prepared using vector D showed higher expression of the codon-converted human anti-WT1 TCR gene with a lower viral copy number, as compared to vector C. In other words, it was shown that expression of the exogenous TCR was high when the configuration of vector D was adopted.

### Example 5: Use of promoter for siRNA expression

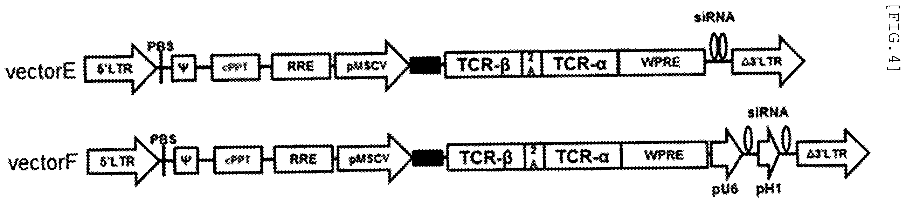

An artificial gene shown by SEQ ID NO: 28 was synthesized. The artificial gene is transcribed into two types of single-stranded RNAs that form stem-loop structures. These single-stranded RNAs produce siRNAs that target nucleotide sequences shown by SEQ ID NOs: 1 and 7 respectively in cells to suppress the expression of the wild-type TCR α-chain and wild-type TCR β-chain genes. Furthermore, an artificial gene shown by SEQ ID NO: 29 was synthesized. The artificial gene contains a human U6 promoter and a human H1 promoter upstream of the siRNAs in order to be transcribed into the siRNAs. Then, these artificial genes were inserted between WPRE and 3' LTR to prepare vector E and vector F as shown in FIG. 4. Virus solutions E and F were prepared in the same manner as in Example 3.

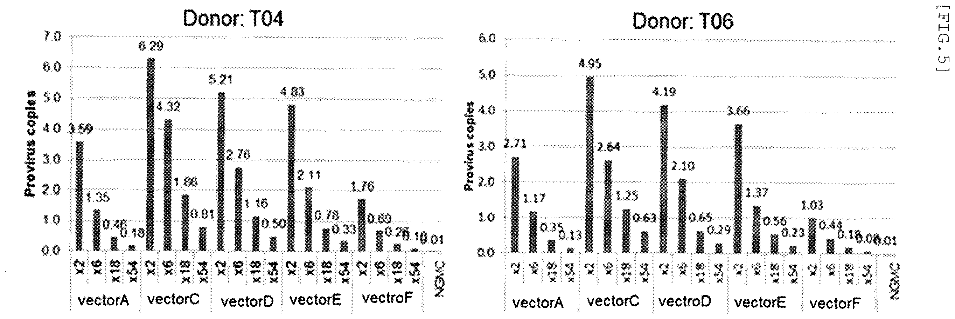

In the same manner as in Example 4, PBMCs obtained with informed consent were infected with virus solutions A, C, D, E, and F, and the number of virus copies integrated into the genome was measured. In addition, the expression levels of the wild-type TCR chain genes and the codon-converted TCR chain genes were measured and their relative values were calculated. The number of virus copies integrated into the genome is shown in FIG. 5. As shown in FIG. 5, the cells infected with vector F had a low copy number. It was shown that when the vector containing promoter sequences between WPRE and 3' LTR was used, the number of viral copies integrated into a genome was low.

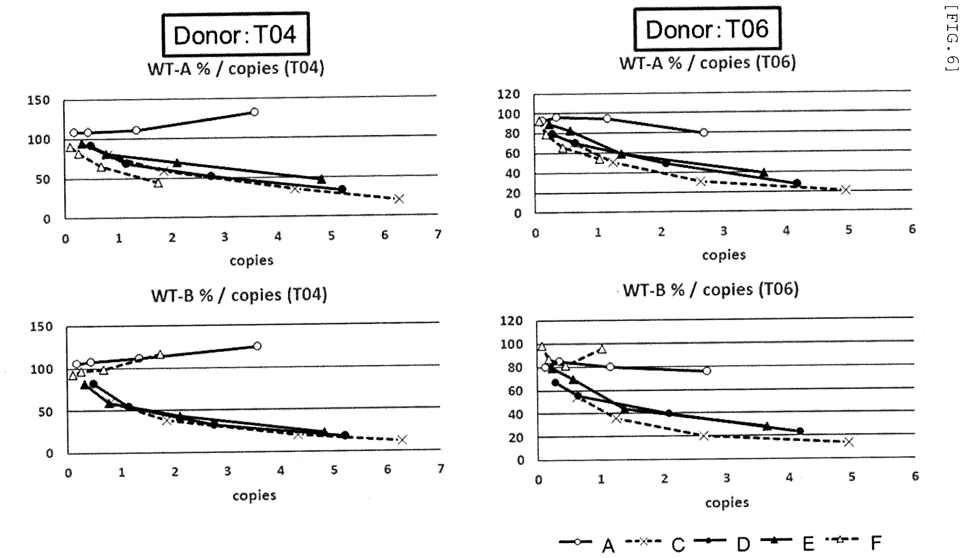

In addition, suppressive effects on the wild-type TCR genes were evaluated by calculating the ratios of values of gene expression in each experimental group relatively to gene expression of the wild-type TCR α-chain and the wild-type TCR β-chain in virus non-infected control cells. Results are shown in FIG. 6. In the figure, the vertical axis shows relative values of gene expression levels when the expression level of the control vector is defined as 100. The horizontal axis shows the number of virus copies. As shown in FIG. 6, vector F did not suppress the expression of the wild-type β-chain gene. In other words, it was shown that the vector containing promoter sequences between WPRE and 3' LTR unstably suppressed the gene expression.

### Industrial Applicability

According to the present invention, provided are a nucleic acid construct that efficiently expresses a gene of interest, a retroviral vector for introducing the nucleic acid construct into cells, a method for producing a gene-introduced cell using the vector, and a cell into which the vector has been introduced. The nucleic acid construct, the retroviral vector, the method for producing a gene-introduced cell and the gene-introduced cell are extremely useful for protein production, treatment of diseases by cell therapy, and research and tests for such protein production and treatment.

### Sequence Listing Free Text

SEQ ID NO:1: A part of wild type TCR alpha chain coding sequence
SEQ ID NO:2: A part of codon modified TCR alpha chain coding sequence
SEQ ID NO:3: A part of TCR alpha chain amino acid sequence SEQ ID NO:4: A part of wild type TCR alpha chain coding sequence
SEQ ID NO:5: A part of codon modified TCR alpha chain coding sequence
SEQ ID NO:6: A part of TCR alpha chain amino acid sequence
SEQ ID NO:7: A part of wild type TCR beta chain coding sequence
SEQ ID NO:8: A part of codon modified TCR beta chain coding sequence
SEQ ID NO:9: A part of TCR beta chain amino acid sequence
SEQ ID NO:10: A part of wild type TCR beta chain coding sequence
SEQ ID NO:11: A part of codon modified TCR beta chain coding sequence
SEQ ID NO:12: A part of TCR beta chain amino acid sequence
SEQ ID NO:13: siRNA generating sequence for wild type TCR genes
SEQ ID NO:14: MSCV U3 promoter region.
SEQ ID NO:15: EF1 alpha SD/SA amplification primer
SEQ ID NO:16: EF1 alpha SD/SA amplification primer
SEQ ID NO:17: P2A peptide coding sequence
SEQ ID NO:18: wild type TCR alpha chain amplification primer F
SEQ ID NO:19: wild type TCR alpha chain amplification primer R
SEQ ID NO:20: wild type TCR beta chain amplification primer F
SEQ ID NO:21: wild type TCR beta chain amplification primer R
SEQ ID NO:22: codon modified TCR alpha chain amplification primer F
SEQ ID NO:23: codon modified TCR alpha chain amplification primer R
SEQ ID NO:24: codon modified TCR beta chain amplification primer F
SEQ ID NO:25: codon modified TCR beta chain amplification primer R
SEQ ID NO:26: GAPDH amplification primer F
SEQ ID NO:27: GAPDH amplification primer R
SEQ ID NO:28: siRNA generating sequence for wild type TCR genes
SEQ ID NO:29: siRNA generating sequence for wild type TCR genes

## Claims

1. A nucleic acid construct for expressing a gene of interest, comprising: in order from the 5' end to 3' end,
(a) a 5' LTR (Long Terminal Repeat) sequence derived from a retrovirus,
(b) a packaging signal sequence (ψ) derived from a retrovirus,
(c) a sequence of the gene of interest or a multiple cloning site,
(d) a posttranscriptional regulatory element (PRE),
(e) a siRNA-producing sequence which is transcribed into an RNA that forms at least one stem-loop structure and induces RNA interference in a mammalian cell, and
(f) a 3' LTR sequence derived from a retrovirus.

2. The nucleic acid construct according to claim 1, wherein the PRE is a PRE derived from Woodchuck hepatitis virus (WPRE).

3. The nucleic acid construct according to claim 1, which comprises a foreign promoter sequence on the 5' side of the gene of interest sequence.

4. The nucleic acid construct according to claim 1, wherein the gene of interest is a gene encoding a T cell receptor (TCR) or a chimeric antigen receptor (CAR).

5. The nucleic acid construct according to claim 4, wherein the gene of interest is a gene encoding a TCR α chain and a TCR β chain linked with a 2A peptide.

6. The nucleic acid construct according to claim 1, wherein the siRNA-producing sequence is a sequence for producing siRNA that acts on an mRNA encoding a constant region of a wild-type TCR to suppress its expression.

7. The nucleic acid construct according to claim 6, wherein the gene of interest is a gene encoding a mutated TCR in which a mutation is introduced into a nucleotide sequence of the constant region, and expression of the gene encoding the mutated TCR is not suppressed by the siRNA.

8. The nucleic acid construct according to claim 1, wherein the siRNA-producing sequence is a sequence for producing siRNA that acts on an mRNA encoding a molecule that suppresses an activity of an immune cell to suppress its expression.

9. A retroviral vector comprising a transcript from the nucleic acid construct according to any one of claims 1-8.

10. The retroviral vector according to claim 9, which comprises a 5' LTR derived from an oncoretrovirus or a lentivirus, a packaging signal sequence derived from an oncoretrovirus or a lentivirus, and a 3' LTR derived from an oncoretrovirus or a lentivirus.

11. A method for producing a retrovirus vector, the method comprising a step of introducing the nucleic acid construct according to any one of claims 1 to 8 into a cell capable of producing a retrovirus particle.

12. A method for producing a gene-introduced cell, the method comprising a step of introducing the retroviral vector according to claim 9 or 10 into a cell.

13. The method according to claim 12, wherein the cell is a T cell, a cell capable of differentiating into a T cell, or a cell population containing the T cell or the cell capable of differentiating into a T cell.

14. A cell containing the nucleic acid construct according to any one of claims 1 to 8.

15. The gene-introduced cell according to claim 14, wherein the cell is a T cell, a cell capable of differentiating into a T cell, or a cell population containing the T cell or the cell capable of differentiating into a T cell.


SEQUENCE LISTING

<110>  TAKARA BIO INC.

<120>  siRNA expression vector

<130>  675854

<150>  JP2019-187309
<151>  2019-10-11

<160>  29

<170>  PatentIn version 3.1

<210>  1
<211>  21
<212>  DNA
<213>  Artificial

<220>
<223>  A part of wild type TCR alpha chain coding sequence

<400>  1
agtaaggatt ctgatgtgta t                                                21


<210>  2
<211>  21
<212>  DNA
<213>  Artificial

<220>
<223>  A part of codon modified TCR alpha chain coding sequence

<400>  2
agcaaggaca gcgacgtgta c                                                21


<210>  3
<211>  7
<212>  PRT
<213>  Artificial

<220>
<223>  A part of TCR alpha chain amino acid sequence

<400>  3

Ser Lys Asp Ser Asp Val Tyr
1               5


<210>  4
<211>  21
<212>  DNA
<213>  Artificial

<220>
<223>  A part of wild type TCR alpha chain coding sequence

<400>  4
agcaacaaat ctgactttgc a                                                21

<210>  5
<211>  21
<212>  DNA
<213>  Artificial

<220>
<223>  A part of codon modified TCR alpha chain coding sequence

<400>  5
tccaacaaga gcgacttcgc c                                                21


<210>  6
<211>  7
<212>  PRT
<213>  Artificial

<220>
<223>  A part of TCR alpha chain amino acid sequence

<400>  6

Ser Asn Lys Ser Asp Phe Ala
1               5


<210>  7
<211>  21
<212>  DNA
<213>  Artificial

<220>
<223>  A part of wild type TCR beta chain coding sequence

<400>  7
gccaccatcc tctatgagat c                                                21


<210>  8
<211>  21
<212>  DNA
<213>  Artificial

<220>
<223>  A part of codon modified TCR beta chain coding sequence

<400>  8
gccaccatcc tgtacgagat c                                                21


<210>  9
<211>  7
<212>  PRT
<213>  Artificial

<220>
<223>  A part of TCR beta chain amino acid sequence

<400>  9

Ala Thr Ile Leu Tyr Glu Ile

1               5


<210>  10
<211>  21
<212>  DNA
<213>  Artificial

<220>
<223>  A part of wild type TCR beta chain coding sequence

<400>  10
gccatggtca agagaaagga t                                                21


<210>  11
<211>  21
<212>  DNA
<213>  Artificial

<220>
<223>  A part of codon modified TCR beta chain coding sequence

<400>  11
gccatggtga agcggaagga c                                                21


<210>  12
<211>  7
<212>  PRT
<213>  Artificial

<220>
<223>  A part of TCR beta chain amino acid sequence

<400>  12

Ala Met Val Lys Arg Lys Asp
1               5


<210>  13
<211>  630
<212>  DNA
<213>  Artificial

<220>
<223>  siRNA generating sequence for wild type TCR genes

<400>  13
tatttcaaat ttagcaggaa aaaagagaac atcaccttgt aaaactgaag attgtgacca      60

gtcagaataa tgtgtaagga ttctgatgtg tacacaggga agcgagtctg tacacatcag     120

aatccttaca gcattatggt gacagctgcc tcgggaagcc aagttgggct ttaaagtgca     180

gggcctgctg atgttgagtg ctttttgttc ccaccatcct ctatgagatt agtgctcctg     240

gttgatctca tagaggatgg tggggcataa gaagttatgt attcatccaa taattcaagc     300

caagcaagta tataggtgtt ttaatagttt ttgtttgcag tcctctgttc catggtcaag     360

agaaaggaca tgctacagtc aagatgtcct ttctcttgac catggtggtg gcctgctatt     420

tccttcaaat gaatgatttt tactaatttt gtgtactttt attgtgtcga tgtagaatct 480

gcctggtcta tctgatgtga cagcttctgt agcaccaaca aatctgactt tgcaactgtg 540

ctctgagaag ctgcaaagtc agatttgttg tactgctagc tgtagaactc cagcttcggc 600

ctgtcgccca atcaaactgt cctgttactg 630

<210> 14
<211> 366
<212> DNA
<213> Artificial

<220>
<223> MSCV U3 promoter region.

<400> 14
agcttaagta acgccatttt gcaaggcatg gaaaatacat aactgagaat agagaagttc 60

agatcaaggt taggaacaga gagacagcag aatatgggcc aaacaggata tctgtggtaa 120

gcagttcctg ccccggctca gggccaagaa cagatggtcc ccagatgcgg tcccgccctc 180

agcagtttct agagaaccat cagatgtttc cagggtgccc caaggacctg aaatgaccct 240

gtgccttatt tgaactaacc aatcagttcg cttctcgctt ctgttcgcgc gcttctgctc 300

cccgagctca ataaaagagc ccacaacccc tcactcggcg cgccagtcct ccgatagact 360

gcgtcg 366

<210> 15
<211> 20
<212> DNA
<213> Artificial

<220>
<223> EF1 alpha SD/SA amplification primer

<400> 15
ctttttcgca acgggtttgc 20

<210> 16
<211> 30
<212> DNA
<213> Artificial

<220>
<223> EF1 alpha SD/SA amplification primer

<400> 16
tttggctttt aggggtagtt ttcacgacac 30

<210> 17
<211> 57
<212> DNA
<213> Artificial

<220>
<223>  P2A peptide coding sequence

<400>  17
gccaccaact tcagcctgct gaagcaggcc ggtgacgtcg aggagaatcc tggcccc        57


<210>  18
<211>  21
<212>  DNA
<213>  Artificial

<220>
<223>  wild type TCR alpha chain amplification primer F

<400>  18
gtgcaaacgc cttcaacaac a                                               21


<210>  19
<211>  23
<212>  DNA
<213>  Artificial

<220>
<223>  wild type TCR alpha chain amplification primer R

<400>  19
gaccagcttg acatcacagg aac                                             23


<210>  20
<211>  22
<212>  DNA
<213>  Artificial

<220>
<223>  wild type TCR beta chain amplification primer F

<400>  20
cgccctcaat gactccagat ac                                              22


<210>  21
<211>  20
<212>  DNA
<213>  Artificial

<220>
<223>  wild type TCR beta chain amplification primer R

<400>  21
cctgggtcca ctcgtcattc                                                 20


<210>  22
<211>  21
<212>  DNA
<213>  Artificial

<220>
<223>  codon modified TCR alpha chain amplification primer F

<400>  22
agagcttcga gaccgacacc a                                                21


<210>  23
<211>  19
<212>  DNA
<213>  Artificial

<220>
<223>  codon modified TCR alpha chain amplification primer R

<400>  23
tgaagccggc cactttcag                                                   19


<210>  24
<211>  20
<212>  DNA
<213>  Artificial

<220>
<223>  codon modified TCR beta chain amplification primer F

<400>  24
ggcttcacca gcgagagcta                                                  20


<210>  25
<211>  18
<212>  DNA
<213>  Artificial

<220>
<223>  codon modified TCR beta chain amplification primer R

<400>  25
tcaccatggc catcagca                                                    18


<210>  26
<211>  20
<212>  DNA
<213>  Artificial

<220>
<223>  GAPDH amplification primer F

<400>  26
gcaccgtcaa ggctgagaac                                                  20


<210>  27
<211>  19
<212>  DNA
<213>  Artificial

<220>
<223>  GAPDH amplification primer R

<400>  27
tggtgaagac gccagtgga                                                   19

<210>  28
<211>  327
<212>  DNA
<213>  Artificial

<220>
<223>  siRNA generating sequence for wild type TCR genes

<400>  28
tatttcaaat ttagcaggaa aaaagagaac atcaccttgt aaaactgaag attgtgacca      60

gtcagaataa tgtgtaagga ttctgatgtg tacacaggga agcgagtctg tacacatcag     120

aatccttaca gcattatggt gacagctgcc tcgggaagcc aagttgggct ttaaagtgca     180

gggcctgctg atgttgagtg ctttttgttc ccaccatcct ctatgagatt agtgctcctg     240

gttgatctca tagaggatgg tggggcataa gaagttatgt attcatccaa taattcaagc     300

caagcaagta tataggtgtt ttaatag                                         327


<210>  29
<211>  528
<212>  DNA
<213>  Artificial

<220>
<223>  siRNA generating sequence for wild type TCR genes

<400>  29
gagggcctat ttcccatgat tccttcatat ttgcatatac gatacaaggc tgttagagag      60

ataattagaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaggatccg taaggattct gatgtgtaca cagggaagcg agtctgtaca catcagaatc     300

cttacttttt tgcatgcaag cttgatatcg atatcgaatt catttgcatg tcgctatgtg     360

ttctgggaaa tcaccataaa cgtgaaatgt ctttggattt gggaatctta taagttctgt     420

atgagaccac tcggatccgc caccatcctc tatgagatta gtgctcctgg ttgatctcat     480

agaggatggt ggcttttttg catgcaagct tgatatcgat agcttggc                  528